**Europäisches Patentamt**

**European Patent Office**

**Office européen des brevets**

(19)

(11) Veröffentlichungsnummer : **0 287 920 B1**

(12)

# EUROPÄISCHE PATENTSCHRIFT

(45) Veröffentlichungstag der Patentschrift :
**15.05.91 Patentblatt 91/20**

(51) Int. Cl.$^5$ : **A61M 5/14, F04B 13/00**

(21) Anmeldenummer : **88105739.2**

(22) Anmeldetag : **11.04.88**

(54) **Kolbenpumpe für ein Medikamentendosiergerät.**

(30) Priorität : **22.04.87 DE 3713498**

(43) Veröffentlichungstag der Anmeldung :
**26.10.88 Patentblatt 88/43**

(45) Bekanntmachung des Hinweises auf die
Patenterteilung :
**15.05.91 Patentblatt 91/20**

(84) Benannte Vertragsstaaten :
**DE FR GB IT SE**

(56) Entgegenhaltungen :
**EP-A- 0 103 536**
**DE-A- 3 515 848**
**US-A- 3 468 257**
**US-A- 4 543 989**

(73) Patentinhaber : **Siemens Aktiengesellschaft**
**Wittelsbacherplatz 2**
**W-8000 München 2 (DE)**

(72) Erfinder : **Buchholtz, Gerhard, Dipl.-Ing.**
**Im Heuschlag 26**
**W-8520 Erlangen (DE)**
Erfinder : **Franetzki, Manfred, Dr.**
**Schleifweg 7B**
**W-8525 Uttenreuth (DE)**
Erfinder : **Obermann, Peter, Dipl.-Ing.**
**Am Färberhof 12**
**W-8520 Erlangen (DE)**
Erfinder : **Fickweiler, Werner**
**Im Heuschlag 27**
**W-8520 Erlangen (DE)**
Erfinder : **Geisselbrecht, Georg, Dipl.-Ing.**
**Riemenschneider Strasse 16**
**W-8520 Erlangen (DE)**

**Beschreibung**

Die Erfindung betrifft eine Kolbenpumpe nach dem Oberbegriff des Patentanspruchs 1.

Bei einem implantierbaren Medikamentendosiergerät, z.B. bei einem Insulin-Dosiergerät, bei dem ein empfindliches flüssiges Medikament in kleinen Raten gefördert wird, ist es aus Gründen der Miniaturisierung vorteilhaft, als Pumpaggregat eine miniaturisierte Kolbenpumpe einzusetzen. Der in diesem Anwendungsfall benötigte Hubraum einer solchen Pumpe liegt z.B. in der Größenordnung von 0,5 bis 1 µl.

Die Kolbenpumpe sollte in der Lage sein, auch Gasblasen mit einer zufriedenstellenden Förderrate zu pumpen, damit bei Auftreten einer Gasblase an irgendeiner Stelle im Fluidtrakt die Förderung des Medikaments nicht aufhört und der Patient nicht längere Zeit unversorgt bleibt. Eine Gasblase im Medikamentenspeicher entsteht beispielsweise durch Ausgasen des Medikaments oder beim Nachfüllen des Medikamentenspeichers. Das Problem der Gasblasenförderung stellt sich noch verstärkt bei einem Medikamentendosiergerät, bei dem der Medikamentenspeicher mit einem Unterdruck beaufschlagt ist (DE-C-26 52 026). Ein solcher sicherheitstechnisch vorteilhafter Unterdruck im Medikamentenspeicher liegt in einem typischen Fall zwischen 0,5 und 1 bar absolut. Dadurch ergibt sich aus physikalischen Gründen bei einer idealen Pumpe mit totraumfreier Pumpkammer eine Reduzierung der Gasförderrate maximal um den Faktor 2 gegenüber der Flüssigkeitsförderrate.

Hat die Pumpe dagegen einen Totraum in der Pumpkammer, der die gleiche Größe wie der Hubraum aufweist, ist aus physikalischen Gründen an der unteren Grenze des Unterdruckes (0,5 bar absolut) im Speichersystem eine Förderung von Gasblasen völlig unmöglich. Eine in der Pumpkammer befindliche Gasblase würde lediglich komprimiert und dekomprimiert werden, ohne daß ein Transport zur Auslaßseite stattfindet. Eine zuverlässige Funktion ist auch bei geringeren Unterdrücken nicht mehr gewährleistet. Dieses Problem stellt sich in der Regel nicht bei größeren Pumpen mit einem Hubraum über 10 µl.

Das genannte Problem wird nicht nur akut, wenn ein abgeschlossener Unterdruckspeicher, der bei einem Insulin-Infusionsgerät einen Unterdruck von 300 bis 500 mbar (500 bis 700 mbar absolut) aufweist, an die Pumpe angeschlossen ist. Dasselbe Problem tritt auf, wenn gegen einen Überdruck gefördert wird, z.B. gegen einen fast vollständig verstopften Katheter. Allgemein formuliert kann man sagen : Besteht eine nennenswerte Differenz zwischen (niederem) Eingangsdruck und (höherem) Ausgangsdruck an der Pumpe, besteht die Gefahr, daß trotz Aufrechterhaltung der Kolbenbewegung kein Medikament gefördert wird ; diese Differenz kann dabei durch einen Unterdruck am Medikamentenspeicher oder durch einen Überdruck am Katheter zustandekommen.

Aus den genannten Gründen ist es anzustreben, bei einer Kolbenpumpe ein möglichst großes Verhältnis von Hubraum zu Totraum (Verdichtungsverhältnis) in der Pumpkammer zu erreichen. Der Erfindung liegt folgende Überlegung zugrunde : Da bei einem Medikamentendosiergerät, insbesondere bei einem implantierbaren Gerät, kleine Flüssigkeitsmengen zu fördern sind, ist eine Vergrößerung des Hubraumes der Kolbenpumpe in der Regel nicht möglich. Der Ansatzpunkt für die Erzielung einer großen Gasförderrate liegt also in der Reduzierung oder gar Vermeidung des Totraumes.

Eine Kolbenpumpe der eingangs genannten Art ist aus der US-A- 4 568 250 bekannt. Dort ist eine Einlaßkammer vorgesehen, welche im wesentlichen von einer Gehäusestirnfläche mit einer Öffnung für eine Einlaßleitung, einer zylindrischen Wand und der Stirnfläche eines Kolbens gebildet wird. Der Kolben befindet sich – unter Bildung eines Ringspaltes – in einem Zylindergehäuse. Innerhalb der Einlaßkammer ist ein bewegliches Ventilteil als Teil eines Rückschlagventils angeordnet. Ein mechanisches Federsystem hält das Ventilteil in einer Ruhestellung gegen die Einlaßöffnung gedrückt. Durch das Federsystem mit zugehörigen Befestigungsmitteln entsteht ein relativ großer Totraum, der sich ungünstig auf die Gasförderung der Kolbenpumpe auswirkt.

Aus der US-A-3 468 257 ist eine weitere Kolbenpumpe des vorbeschriebenen Typs bekannt, deren Ventilteil in Form eines Rückschlagventils an der Auslaßseite der Pumpe angeordnet ist. Auch dieses Ventil wird von einem mechanischen Federsystem betätigt, welches einen verhältnismäßig großen Totraum für die Flüssigkeit benötigt, der bei einem eventuellen Auftreten von Gasblasen in der zu fördernden Flüssigkeit zu Betriebsstörungen führt.

Aus der DE-A-35 15 848 ist ein Ventil für Medikamentendosiergeräte bekannt, welches nicht als Bestandteil solcher Dosiergeräte, sondern als zusätzliches Element für die Einordnung in eine Zu- oder Ableitung der zu transportierenden Flüssigkeit vorgesehen ist. Bei diesem Ventil wird die Schließkraft des Schließkörpers zwar auch magnetisch erzeugt, jedoch ist wegen der besonderen Ausgestaltung des Schließkörpers des Ventils als Kugelverschluß, der an einem elastischen O-Ring anliegt, auch wenn dieses Ventil in ein Medikamentendosiergerät integriert werden sollte, ein dichtes Anlegen an den Kolben des Dosiergerätes nicht möglich, so daß auch in diesem Fall ein relativ großer Totraum entstünde, der sich bei auftretenden Gasblasen in der zu fördernden Flüssigkeit in der beschriebenen Weise ungünstig auswirken würde.

Aus der EP-A-0 103 536, insbesondere Fig. 3, ist gleichfalls eine Kolbenpumpe der eingangs genannten Art bekannt. Auch hier handelt es sich um eine Kolbenpumpe mit eingebautem Rückschlagventil, welches hier jedoch ausgangsseitig angeordnet ist. Auch bei dieser Kolbenpumpe ist ein in einem Zylinder ohne dichtenden Abschluß zur Zylinderwandung beweglicher Kolben derart angeordnet, daß er durch eine Bewegung in einer ersten Richtung das zu fördernde Medium in Förderrichtung verschiebt, womit ein Ansaugen aus einem Medikamentenreservoir verbunden ist. Die Kolbengeschwindigkeit ist so groß gewählt, daß das Medium bei Vorhandensein eines Gegendrucks nicht vollständig durch den Spalt zwischen Kolben und Zylinder abfließt. Bei einer Bewegung in einer zweiten Richtung, die der ersten entgegengerichtet ist, wird die Pumpkammer durch einen Medienfluß durch den Spalt zwischen Kolben und Zylinderwand wieder aufgefüllt. Bei dieser Konstruktion ist das Rückschlagventil durch eine federnde Membran, die einen längeren Kanal abschließt, gebildet. Auch hier sind Schwierigkeiten und Funktionsstörungen beim Pumpen zu befürchten, sobald Gasblasen im Fördermedium enthalten sind.

Aufgabe der Erfindung ist es, eine Kolbenpumpe der eingangs genannten Art anzugeben, welche in ihrer Zuverlässigkeit unempfindlich gegen das Auftreten von Gasblasen ist und welche durch Verringerung des Totraumes eine hohe Gasförderrate ermöglicht.

Diese Aufgabe wird durch die im Patentanspruch 1 angegebene Erfindung gelöst.

Bevorzugt ist die Dichtfläche dünn ausgebildet. Sind die Dichtfläche und die Stirnseite des Kolbens ideal plane Flächen, so könnte es sich ergeben, daß sich diese Flächen beim Übergang von der Hub- in die Senkstellung nicht voneinander lösen. Um diese Schwierigkeit zu umgehen, ist nach einer Ausbildung vorgesehen, daß entweder die eine, die andere oder beide Flächen leicht aufgerauht ist/sind. Eine leichte punktuelle Aufrauhung kann beispielsweise durch eine Vertiefung in einer oder beider Flächen, z.B. in Form eines Ringspalts, einer Nut oder Rille erreicht werden. Auch eine Erhebung, ein Dorn, ein kleiner Stift etc. an zumindest einer Fläche kann demselben Zweck dienen.

Eine bevorzugte Weiterbildung zeichnet sich dadurch aus, daß der Hub des Kolbens größer bemessen ist als der Abstand zwischen der Stirnseite des Kolbens in seiner Ruhelage und der Dichtfläche.

Der Begriff "eng anliegend" bedeutet im Rahmen der vorliegenden Erfindung, daß ein flächiges Berühren, ggf. unter Zwischenschaltung einer kleineren oder größeren Aufrauhung (Rille, Erhebung, etc.) stattfindet. Es kann daher auch ein dünnes Element (Schicht, Platte) mit Durchlaßöffnung, z.B. in Form einer aus der Optik an sich bekannten Lochblende, zwischen der Endposition des Zylinders und der Dichtfläche angeordnet sein.

Durch die Anordnung des Ventilteiles am Auslaß und durch ggf. gleichzeitige Anpassung der Dichtfläche des Ventilteiles an die Gestaltung der Stirnseite des Kolbens mit der Folge, daß in Hubstellung die Dichtfläche eng an der Stirnseite "anliegt", wird erreicht, daß nahezu totraumfrei Flüssigkeit gefördert werden kann. In Hubstellung des Kolbens befindet sich zwischen dessen Stirnseite und der Dichtfläche aufgrund des engen Anliegens weder Flüssigkeit noch Luft. Beim Zurückgleiten des Kolbens in Richtung auf seine Senkstellung bleibt die Dichtfläche des Ventilteils eng an der Stirnseite des Kolbens angelegt, bis die Dichtfläche an einen Anschlag, z.B. an eine Ringschneide, gelangt. Wird der Kolben weiter in Richtung auf seine Senkstellung bewegt, kann sich die Pumpkammer mit aus einem Reservoir über den Spalt zwischen Zylinder und Kolben nachströmender Flüssigkeit füllen. Ein möglicherweise vorhandenes Gas ist in den Auslaß gedrückt worden ; es wird durch die Ringschneide in Verbindung mit der Dichtfläche daran gehindert, in die Pumpkammer zurückzuströmen.

Mit Vorteil wird die Kolbenpumpe in Verbindung mit einem unter Unterdruck stehenden Medikamentenreservoir eingesetzt.

Weitere Vorteile und Ausgestaltungen der Erfindung ergeben sich aus der Beschreibung von Ausführungsbeispielen anhand von drei Figuren. Es zeigen:

Fig. 1 im Querschnitt eine Kolbenpumpe mit strömungsgünstigem Fluidtrakt, mit Pumpkammer, mit Kolben in Ruhestellung und mit am Auslaß angeordnetem Ventil,

Fig. 2 im Querschritt eine Kolbenpumpe mit strömungsgünstigem Fluidtrakt, mit Pumpkammer, mit Kolben in Ruhestellung, mit koaxialem Antriebssystem und mit einem am Auslaß angeordneten, titangekapselten Ventil, und

Fig. 3 im Querschnitt eine Pumpkammer mit Kolben, mit Lochblende und mit am Auslaß angeordneten Ventil.

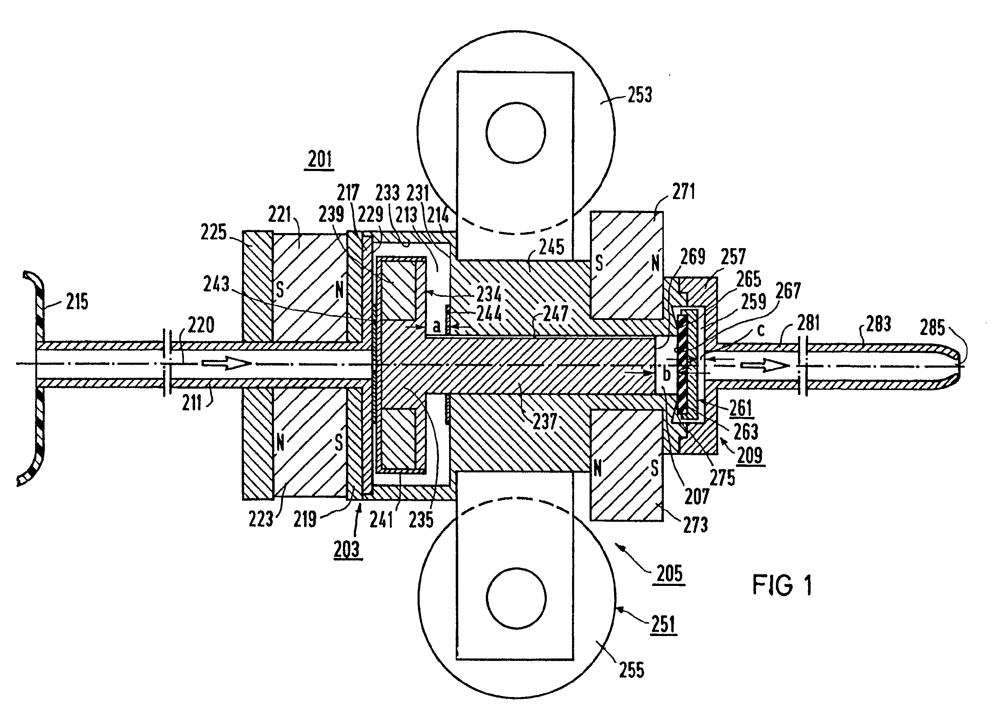

In Fig. 1 ist eine Kolbenpumpe 201 für ein Fördervolumen von weniger als 10 µl, insbesondere für etwa 1 µl, dargestellt. Diese Kolbenpumpe 201 setzt sich im wesentlichen aus einem Einlaßsystem 203 mit magnetischer Rückstellfeder, einem Pumpsystem 205 mit Pumpkammer 207 und einem Auslaßsystem 209 mit integriertem Rückschlagventil zusammen.

Das Einlaßsystem 203 umfaßt einen Fluidtrakt, der eine Einlaßleitung 211 und eine zylindrische Einlaßkammer 213 in einem Gehäuse 214 besitzt. An die Einlaßleitung 211 ist ein Medikamentenreservoir 215 anschließbar, das vorzugsweise mit Unterdruck gegenüber der Umgebung, z.B. mit 300 mbar, beaufschlagt ist. Bei dem Reservoir 215 kann es sich insbesondere um einen Behälter mit Insulin handeln. Die Einlaßleitung 211 mündet über einen Flansch 217 in die Einlaßkammer 213. Letztere weist gegenüber der Einlaßleitung 211 einen wesentlich vergrößerten Querschnitt auf. Alternativ zu der Darstellung ist es auch möglich, auf die Einlaßleitung 211 zu verzichten und die Einlaßkammer 213 als Bestandteil des Medikamentenreservoirs 215 auszuführen.

Auf dem Endstück der Einlaßleitung 211 sitzt ein nicht-magnetisierbarer zylindrischer Tragkörper 219, an dem diametral zur Achse 220 zwei Permanentmagnete 221, 223 angebracht sind. Letztere sind z.B. jeweils von zylindrischer oder quaderförmiger Gestalt und besitzen die eingezeichneten Polaritäten S-N und N-S. Sie können auch durch einen einzigen, z.B. axial angeordneten, Permanentmagneten ersetzt werden. Die Permanentmagnete 221, 223 stellen eine Rückstellkraft für das Pumpsystem 205 bereit (wie später deutlich wird, dienen sie als Federsystem oder Rückstellfeder zum Anziehen des Kolbens 237 in seine Ruhelage). Ein magnetisches Rückschlußteil 225, z.B. aus Weicheisen, das ebenfalls zylindrisch ausgebildet ist, sitzt auf den ventilabgewandten Polen S, N der Magnete 221 bzw. 223.

Die Einlaßkammer 213 wird begrenzt durch die Stirnseite 229 des Flansches 217 sowie durch eine Stirnseite 231 und eine zylindrische Innenwand 233 des Gehäuses 214. In der Einlaßkammer 213 befindet sich ein axial verschiebbarer zylindrischer Anker 234. Dieser besitzt einen Ankerträger 235, der am rechten Ende in einen längeren Kolben 237 geringeren Durchmessers übergeht. Abweichend davon kann der Ankerträger 235 auch durch Befestigungsmittel an dem Kolben 237 befestigt sein.

Im gezeigten Ausführungsbeispiel ist auf dem Ankerträger 235 ein ringförmiges Ankerteil 239 befestigt. Das Ankerteil 239 ist dabei über den Kern des in den Kolben 237 übergehenden Ankerträgers 235 gestülpt. Das Ankerteil 239 ist aus einem magnetisierbaren Material, beispielsweise Weicheisen, gefertigt. Der Ankerträger 235 ist teilweise und das Ankerteil 239 ist vollständig von einer zylindrischen Dose oder Kapsel 241 umgeben oder eingefaßt. Um eine Wechselwirkung mit dem durch die Kolbempumpe 201 zu fördernden flüssigen Medikament zu vermeiden, ist also die Kapsel 241 so um das Ankerteil 239 gelegt und mit dem Ankerträger 235 randseitig verbunden, daß eine vollständige Kapselung des Ankerteiles 239 erzielt wird.

Die außerhalb der Einlaßkammer 213 plazierten Permanentmagnete 221, 223 üben auf den Anker 234 dauernd eine Kraft aus, die den Anker 234 samt Kolben 237 in Richtung auf die Einlaßleitung 211 hinzieht, die Einlaßöffnung im Flansch 217 dabei aber nicht verschließt. Für die Begrenzung dieser Rückziehbewegung kann ein Anschlag 243, z.B. in Form einer dünnen ringförmigen und zentral befestigten Platte, an der Stirnseite 229 des Flansches 217 vorgesehen sein. Ein weiterer Anschlag 244, z.B. ebenfalls in Form eines dünnen, axial plazierten Ringes, kann auf der anderen Stirnseite 231 der Einlaßkammer 213 angebracht sein. Befindet sich der Anker 234 an der linken Stirnseite 229, so ist der Kolben 237 in seiner Senkstellung oder Ruhelage ; der Abstand zwischen der Rückseite des Ankers 234 und der Stirnseite 231 oder ggf. dem Anschlag 244 ist dann genau definiert und beträgt a.

Die beiden Permanentmagnete 221, 223 samt Halterung 219 und Rückschlußteil 225 sind vorzugsweise in Richtung der Längsachse 220 definiert verschiebbar, wodurch die Rückstellkraft auf den Anker 234 und damit den Kolben 237 kontinuierlich auf einen gewünschten Wert einstellbar ist.

Der Kolben 237 ist longitudinal im hinteren Teil des Gehäuses 214, der als Zylinder 245 dient, verschiebbar. Zwischen Zylinder 245 und Kolben 237 ist dabei ein Spalt 247, insbesondere ein Ringspalt, vorgesehen, durch den bei Rückstellung des Kolbens 237 in seine Ruhelage das flüssige Medikament von links nach rechts gefördert wird, wie später näher dargelegt ist.

Der Anker 234 mit ferromagnetischem Ankerteil 239 dient gleichzeitig auch als Anker für ein elektromagnetisches Antriebssystem 251, das am Zylinder 245 angeordnet ist. Dieses weist zwei elektromagnetische Spulen 253, 255 auf. Bei deren Erregung mittels eines Stromes wird ein magnetisches Feld erzeugt, das eine ausreichend große Kraftwirkung auf den Anker 234 bereitstellt. Dadurch wird der Anker 234 samt Kolben 237 in Richtung auf das Auslaßsystem 209 verschoben, und zwar bis der Anker 234 an der rechten Stirnseite 231 oder an dem Anschlag 244 anstößt.

Stromabwärts direkt hinter der Pumpkammer 207 befindet sich – teils im Gehäuse 214, teils in einem Ansatzflansch 257 – eine Auslaßkammer 259. In dieser Auslaßkammer 259 ist ein bewegliches zylindrisches Ventilteil 261 untergebracht. Dieses ist Teil des genannten Rückschlagventils. Das Ventilteil 261 hat einen größeren Durchmesser als der Kolben 237. Es ist hier zweistückig ausgebildet und besitzt ein schalenförmiges Schließelement 263, auf dem kolbenseitig eine Dichtungsscheibe 265 mit einer Dichtfläche 267 befestigt ist. Die Dichtungsscheibe 265 kann insbesondere aus einem inerten Kunststoff bestehen.

Die Pumpkammer 207 wird stromabwärts von dem axialen Bereich der Dichtfläche 267 begrenzt. Sie wird weiter durch die Zylinderinnenfläche des Gehäuses 245 und durch die Stirnseite 269 des Kolbens 237 begrenzt. Bei der gezeigten Senkstellung des Kolbens 237 ist der Abstand zwischen der die Pumpkammer 207 verschließenden Dichtfläche 267 und der Stirnfläche 269 genau definiert und beträgt b. Es gilt : b ist kleiner oder gleich a, wobei a wie oben angegeben definiert ist, d.h.

$$a = b + \Delta b, \quad (1)$$

wobei $\Delta b$ klein ist und z.B. 0 mm oder insbesondere 5/100 mm beträgt. Der Abstand c, der die Bewegungsmöglichkeit des Ventilteils 261 beschreibt, ist etwas größer als Δ b. Bei einem Insulin-Dosiergerät beträgt z.B. c = 0,3 mm.

Das Schließelement 263 des Ventilteils 261 ist aus einem magnetisierbaren Material gefertigt und dient als Anker. Es bildet zusammen mit zwei Dauermagneten 271 und 273, die im Zylinder 245 mit den angegebenen Polaritäten S-N bzw. N-S um die Achse 220 gruppiert und außerhalb der Pumpkammer 207 angeordnet sind, ein magnetisches Federsystem. Anstelle zweier Magnete 271, 273 kann auch ein ringförmiger Dauermagnet verwendet werden. Das magnetische Federsystem 271, 273, 263 hält das Ventilelement 261 in einer Ruhestellung. Dabei ist die Dichtfläche 267 der Dichtscheibe 265 gegen die Pumpkammereinlaßöffnung, die als Ringschneide 275 ausgebildet ist, gedrückt. Beim Anlegen der Dichtfläche 267 an die Ringschneide 275 wird ein flüssigkeits-und gasdichter Verschluß gebildet.

Die im dargestellten Fall in der Ruhestellung des Ventilteils 261 erzielte Andruckkraft der Dichtfläche 267 gegen die Ringschneide 275 kann abweichend auch durch ein mechanisches Federsystem erzeugt werden. Dieses mechanische Federsystem (nicht gezeigt) sollte dann im ausgangsseitigen Teil der Auslaßkammer 259 angeordnet sein. Ein solches Federsystem kann beispielsweise durch eine Spiralfeder gebildet sein, die sich im Bereich zwischen der Rückseite des Teils 263 und der Frontseite des Flansches 257 befindet.

Abweichend von der Darstellung sind die Dauermagnete 271, 273 zweckmäßigerweise entlang der Längsachse 220 des Zylinders 245 definiert verschiebbar angeordnet. Das ermöglicht es, die Rückstellkraft auf das Ventilelement 261 bei fertig montierter Kolbenpumpe 201 nachträglich einzustellen. Diese Einstellmöglichkeit verändert in keiner Weise das Totvolumen der Pumpkammer 207.

An die Auslaßkammer 259 mit Ventilteil 261 schließt über den Ansatz- oder Auslaßflansch 257 eine Auslaßleitung 281 an, die in einen Katheter 283 mit Auslaßöffnung 285 übergeht. Bei der Implantation ist die Auslaßöffnung 285 an vorgegebener Stelle im Patienten positioniert.

Im Betrieb der Kolbenpumpe 201 wird der Kolben 237 durch die elektromagnetische Feldwirkung der Spulen 253, 255 aus der dargestellten Ruhelage nach rechts bewegt, wodurch im Medikament in der Pumpkammer 207 ein starker Überdruck entsteht. Sobald die durch den Überdruck auf das bewegliche Ventilteil 261 ausgeübte Kraft die Rückstellkraft der magnetischen Feder 263, 271, 273 überschreitet, hebt sich das Ventilteil 261 mit seiner Dichtfläche 267 von der Ringschneide 275 ab. Zwischen dem Ventilteil 261 und der Ringschneide 275 bildet sich ein Ventilspalt. Das flüssige Medikament, z.B. Insulin, wird gegen den Katheterdruck und den Öffnungsdruck des Ventilteils 261 über die Auslaßleitung 281 in den Katheter 283 gedrückt. Der Katheter 283 ist dabei entweder direkt an der Auslaßkammer 259 angeschlossen, oder er ist über einen (nicht gezeigten) Stutzen, der von der Auslaßkammer 259 ausgeht, angeschlossen. Das in der Pumpkammer 207 enthaltene flüssige Medikament durchströmt dabei den Ventilspalt, die Auslaßkammer 259 und den Katheter 283 in Pfeilrichtung. Hat der Kolben 237 seine (nicht gezeigte) Hubstellung erreicht, verringert sich durch das ausströmende Medikament der Überdruck in der Pumpkammer 207. Ist die maximale Hubstellung erreicht, liegt die Stirnseite 269 des Kolbens 237 eng an der Dichtfläche 267 des Ventilteils 261 an. "Eng anliegen" soll dabei bedeuten, daß sich zwischen den beiden Flächen 269, 267 weder Medikamenten-Flüssigkeit noch Gasblasen befinden. Infolge der gewählten Dimensionierung a größer/gleich b kann das Ventilteil 261 mit der Dichtungsscheibe 265 dabei um den Betrag Δ b von der Ringschneide 275 abheben. Zumindest eine der beiden Flächen 267, 269 sollte leicht aufgerauht sein, um später ein leichtes Ablösen beim Zurückfahren zu ermöglichen.

Ist dagegen a wenig kleiner oder gleich b gewählt, so geschieht folgendes : Sobald die durch den Überdruck in der Pumpkammer 207 auf das bewegliche Ventilteil 261 ausgeübte Kraft die magnetische Rückstellkraft des Federsystems 263, 271, 273 unterschreitet, senkt sich das bewegbare Ventilteil 261 wieder auf die Ringschneide 275, wodurch der Ventilspalt geschlossen wird.

Wichtig bei dem geschilderten Übergang von der Senkstellung des Kolbens 237 in seine Hubposition ist, daß sich die Stirnseite 269 des Kolbens 237 in der maximalen Hubstellung in Höhe der Dichtfläche 267 (in Ruhestellung) befindet oder diese minimal um dem Betrag Δ b überfährt, so daß die Pumpkammer 207 quasi totraumfrei ist. Dadurch wird bei kleinem Hubvolumen von z.B. 1 μl eine hohe Fördervolumenkonstanz und gleichzeitig eine gute Fähigkeit, auch Luft- oder andere Gasblasen zu fördern, erreicht. Dies gilt auch bei einem Unterdruck im Medikamentenspeicher 215 und/oder einem Überdruck im Katheter 283 (z.B. infolge Verstopfung), gegen den die Pumpe 201 anpumpen muß. Mit anderen Worten : Auch beim Auftreten von Gasblasen im zu fördernden Medikament kommt der Pumpvorgang nicht zum Stillstand.

Wird der Kolben 237 nach Abschalten der Spulen 253, 255 wieder in seine (in Fig. 1 gezeigte) Senkstellung zurückbewegt, so lösen sich die Flächen 267, 269 voneinander, und es wird in der Pumpkammer 207 ein Unterdruck erzeugt. Ist dessen absoluter Wert kleiner als derjenige im Medikamentenspeicher 215, so entsteht ein Druckgefälle. Das flüssige Medikament wird dabei gegen den Unterdruck im Medikamentenspeicher 215 durch den stärkeren Unterdruck in der Pumpkammer 207 in diese hineingesaugt. Dabei durchströmt es den Spalt 247 zwischen dem Kolben 237 und dem Zylinder 245. Ventilteil 261 und Ringschneide 275 wirken dabei als Rückschlagventil.

Nach dem besagten Abschalten der Spulen 253, 255 wird der Anker 234 samt Kolben 237 durch die Permanentmagnete 221, 223, die auf das Ankerteil 239 wirken, in seine Senkstellung oder Ruhelage zurückgeführt. Die Rückführung in die Ruhestellung des Kolbens 237 könnte auch durch ein mechanisches Rückführ- oder Federsystem (nicht gezeigt) realisiert werden.

Vorteil der in Fig. 1 gezeigten Kolbenpumpe 201 ist ihre Fähigkeit, kleine Flüssigkeitsvolumina mit hoher Dosiergenauigkeit aus einem Unterdruck-Reservoir 215 zu fördern. Die Funktionsfähigkeit ist dabei nicht durch auftretende Gasblasen im Medikamententrakt beeinträchtigt. Bevorzugt wird die Kolbenpumpe 201 in einem implantierbaren Insulin-Dosiergerät eingesetzt.

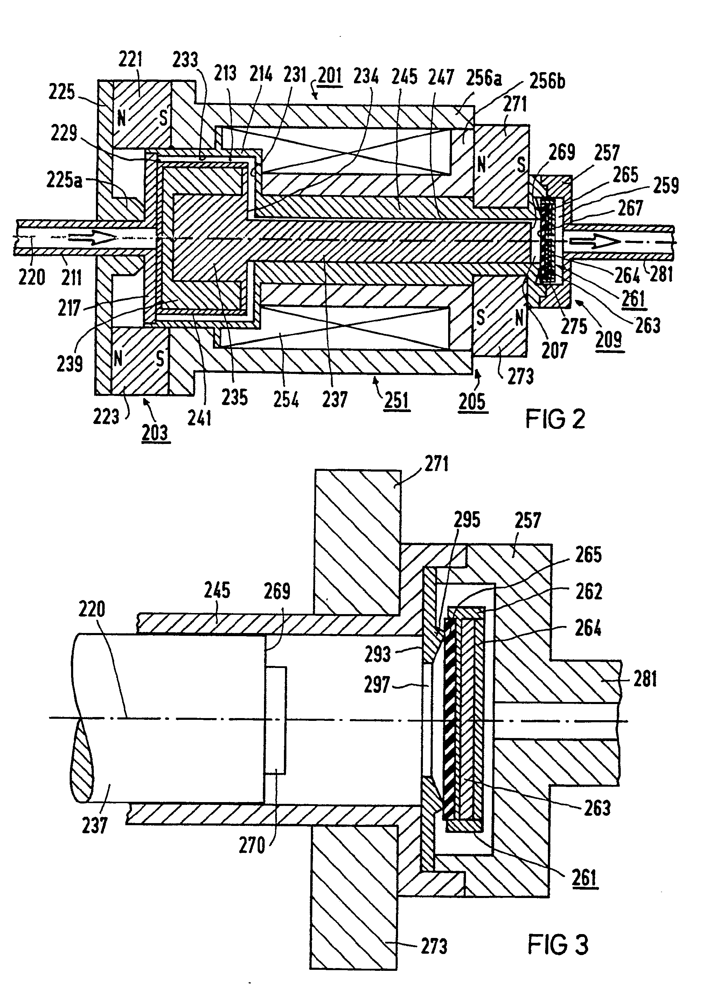

In Fig. 2 ist eine bevorzugte Ausführung einer Kolbenpumpe 201 dargestellt. Gleiche Bauteile sind mit denselben Bezugszeichen versehen wie in Fig. 1. Die Ausführungsformen sind weitgehend gleich. Bemerkenswert ist aber, daß hier ein Tragkörper 219 fehlt. Dafür ist das Rückschlußteil 225 mit einem zentralen rückwärtigen Ansatz 225a versehen. Weiterhin ist hier das Ankerteil 239 topfförmig ausgebildet. Auch werden hier keine Spulen 253, 255 verwendet. Dafür wird hier ein koaxial angeordnetes Antriebssystem 254 in Form eines Elektromagneten verwendet. Dieser ist in einem zweiteiligen Gehäuse 256a, 256b untergebracht, das auf dem Zylinder 245 sitzt. Die Konstruktion ist so gewählt, daß eine einfache Montage möglich ist. Weiterhin ist bemerkenswert, daß beim Ventilteil 261 nach Fig. 2 das Schließelement 263 titangekapselt ist. Weitere Einzelheiten hierzu ergeben sich aus der vergrößerten Darstellung in Fig. 3.

Nach Fig. 3 umfaßt das zylindrische Ventilteil 261 auch hier mehrere Elemente. Ein beidseitig topfförmig ausgebildeter Ankerträger 262, der insbesondere aus Titan gefertigt ist, enthält kolbenseitig eine Dichtungsscheibe 265 und auslaßseitig eine Ankerscheibe 263. Letztere besteht aus einem magnetisierbaren Material. Zum Schutz ist sie allseitig titangekapselt, d.h. sie ist auslaßseitig von einer Abdeckscheibe 264 aus Titan abgedeckt. Letztere ist mit dem Ankerträger 262 gasdicht verschweißt.

Von besonderem Interesse in Fig. 3 ist noch die Anwendung einer Lochblende 293. Diese wird vom Flansch 257 an der Stirnfläche des Zylinders 245 festgehalten. Sie befindet sich also zwischen der Stirnseite 269 und der Dichtfläche 267. Auslaßseitig ist sie konzentrisch mit einem ringförmigen Wulst 295 versehen, um das Abheben des Ventilteils 261 zu erleichtern. Um den Totraum klein zu halten, ist die Stirnseite 269 des Kolbens 237 mit einem Verdränger 270 versehen. Dieser ist zylindrisch ausgebildet und konzentrisch zur Achse 220 angeordnet. Er ist so bemessen, daß er in die Öffnung 297 der Lochblende 295 hineinpaßt.

## Ansprüche

1. Kolbenpumpe für ein Medikamentendosiergerät mit einem Kolben (237), mit einem Fluidtrakt (207, 213, 247), in dessen Auslaß ein bewegbares, eine Dichtfläche aufweisendes Ventilteil (261) als Teil eines Rückschlagventils angeordnet ist, und mit einem auf das Ventilteil in Schließrichtung wirkenden Federsystem (271, 273), **dadurch gekennzeichnet,** daß der Kolben (237) in seiner Hubstellung mit seiner Stirnseite (269) an dem Ventilteil (261) in Bereich dessen Dichtfläche nebene (267) eng anliegt.

2. Kolbenpumpe nach Anspruch 1, **dadurch gekennzeichnet,** daß die Dichtfläche (267) und die Stirnseite (269) des Kolbens (237) eben sind.

3. Kolbenpumpe nach Anspruch 1, **dadurch gekennzeichnet,** daß die Dichtfläche (267) und/oder die Stirnseite (269) des Kolbens (237) aufgerauht oder mit einer Erhebung oder Vertiefung versehen sind/ist.

4. Kolbenpumpe nach einem der Ansprüche 1 bis 3, **dadurch gekennzeichnet,** daß die Dichtfläche (267) in Senkstellung des Kolbens (237) an einer Ringschneide (275) angedrückt ist.

5. Kolbenpumpe nach einem der Ansprüche 1 bis 4, **dadurch gekennzeichnet,** daß das Ventilteil (261) ein Schließelement (263) aus magnetisierbarem Material umfaßt, auf welches mindestens ein am Auslaß (209) angeordneter, vorzugsweise verschiebbarer Permanentmagnet (271, 273) als das Federsystem einwirkt.

6. Kolbenpumpe nach einem der Ansprüche 1 bis 5, **dadurch gekennzeichnet,** daß das Ventilteil (261) eine Dichtscheibe (265) umfaßt, welche mit der Dichtfläche (267) versehen ist.

7. Kolbenpumpe nach einem der Ansprüche 1 bis 6, **dadurch gekennzeichnet,** daß das Ventilteil (261) in einer zylindrischen Auslaßkammer (259) untergebracht ist.

8. Kolbenpumpe nach einem der Ansprüche 1 bis 7, **dadurch gekennzeichnet,** daß der Hub (a) des Kolbens (237) größer bemessen ist als der Abstand (b) zwischen der Stirnseite (269) des Kolbens (237) und der Dichtfläche (267).

9. Kolbenpumpe nach Anspruch 8, **dadurch gekennzeichnet,** daß die Differenz ($\Delta$ b = (a – b)) zwischen Hub (a) und Abstand (b) etwa 5/100 mm beträgt.

10. Kolben nach einem der Ansprüche 1 bis 9, **dadurch gekennzeichnet,** daß der Kolben (237) durch ein elektromagnetisches System (251) in seine Hubstellung überführbar und durch ein Dauermagnetsystem (221, 223, 234) in seine Ruhestellung rückführbar ist.

11. Kolbenpumpe nach einem der Ansprüche 1 bis 10, **gekennzeichnet durch** ihren Einbau in eine implantierbare Medikamenten-Dosiervorrichtung, insbesondere für Insulin.

12. Kolbenpumpe nach einem der Ansprüche 1 bis 11, **dadurch gekennzeichnet,** daß an ihrem Einlaß (203) ein niederer Druck als an ihrem Auslaß (209) herrscht.

13. Kolbenpumpe nach einem der Ansprüche 3 bis 12, **dadurch gekennzeichnet,** daß zwischen der Dichtfläche (267) und der Stirnseite (269) des Kolbens (237) eine Lochblende (293) angeordnet ist (Fig. 3).

14. Kolbenpumpe nach Anspruch 13, **dadurch gekennzeichnet,** daß auf der Stirnseite (269) des Kolbens (237) ein Verdränger (270) angeordnet ist, der in die Öffnung (297) der Lochblende (293) hineinpaßt (Fig. 3).

## Claims

1. Reciprocating pump for a medication dosing apparatus, having a piston (237), and having a fluid passage (207, 213, 247), in the outlet of which there is arranged, as part of a non-return valve, a movable valve part (261) with a sealing surface, and having a spring system (271, 273) acting on the valve part in the direction of closing, **characterised in that** the piston (237), in its lifting position, lies with its front end (269) close against the valve part (261), in the region of the plane of the sealing surface (267) thereof.

2. Reciprocating pump according to claim 1, **characterised in that** the sealing surface (267) and the front end (269) of the piston (237) are level.

3. Reciprocating pump according to claim 1, **characterised in that** the sealing surface (267) and/or the front end (269) of the piston (237) is/are roughened or provided with a bump or a depression.

4. Reciprocating pump according to one of claims 1 to 3, **characterised in that,** in the lowering position of the piston (237), the sealing surface (267) is pressed against a cupped gripping point (275).

5. Reciprocating pump according to one of claims 1 to 4, **characterised in that** the valve part (261) comprises a closing element (263) made of magnetisable material, which is acted upon by at least one permanent magnet (271, 273), preferably displaceable, arranged at the outlet (209), as the spring system.

6. Reciprocating pump according to one of claims 1 to 5, **characterised in that** the valve part (261) comprises a sealing disc (265) which is provided with the sealing surface (267).

7. Reciprocating pump according to one of claims 1 to 6, **characterised in that** the valve part (261) is accommodated in a cylindrical outlet chamber (259).

8. Reciprocating pump according to one of claims 1 to 7, **characterised in that** the length of stroke (a) of the piston (237) is greater than the distance (b) between the front end (269) of the piston (237) and the sealing surface (267).

9. Reciprocating pump according to claim 8, **characterised in that** the difference ($\Delta b = (a - b)$) between the length of stroke (a) and the distance (b) amounts to approximately 5/100 mm.

10. Reciprocating pump according to one of claims 1 to 9, **characterised in that** the piston (237) can be transferred to its lifting position by an electromagnetic system (251) and can be returned to its resting position by a permanent magnet system (221, 223, 234).

11. Reciprocating pump according to one of claims 1 to 10, **characterised by** its installation into an implantable medication dosing apparatus, in particular for insulin.

12. Reciprocating pump according to one of claims 1 to 11, **characterised in that** a lower pressure prevails at its inlet (203) than at its outlet (209).

13. Reciprocating pump according to one of claims 3 to 12, **characterised in that,** between the sealing surface (267) and the front end (269) of the pump (237), a diaphragm (293) is arranged (Figure 3).

14. Reciprocating pump according to claim 13, **characterised in that,** on the front end (269) of the piston (237), there is arranged a displacer (270) which fits into the aperture (297) of the diaphragm (293) (Figure 3).

## Revendications

1. Pompe à piston pour un appareil de dosage de médicaments, comportant un piston (237) et un trajet pour le fluide (207, 213, 247) et dans le conduit de refoulement duquel est montée une pièce de soupape mobile (261), comportant une surface d'étanchéité et faisant partie d'un clapet anti-retour et pourvu d'un système élastique (271, 273) agissant sur la pièce de soupape, dans le sens de la fermeture, caractérisée en ce que le piston (237) s'applique étroitement, en sa position haute, par son côté frontal (269) à la pièce de soupape (261), dans la région du plan de la surface d'étanchéité (267) de celle-ci.

2. Pompe à piston suivant la revendication 1, caractérisée en ce que la surface d'étanchéité (267) et le côté frontal (269) du piston (237) sont plans.

3. Pompe à piston suivant la revendication 1, caractérisée en ce que la surface d'étanchéité (267) et/ou le côté frontal (269) du piston (237) sont rugueux ou sont munis d'une surélévation ou d'une cavité.

4. Pompe à piston suivant l'une des revendications 1 à 3, caractérisée en ce que la surface d'étanchéité (267) est appliquée en position basse du piston (237) contre une arête annulaire (275).

5. Pompe à piston suivant l'une des revendications 1 à 4, caractérisée en ce que la pièce de soupape (261) comprend un obturateur (263) en matériau magnétisable, sur lequel agit au moins un aimant permanent (271, 273), qui est disposé sur le conduit de refoulement (209), qui, de préférence, peut coulisser et qui sert de système plastique.

6. Pompe à piston suivant l'une des revendications 1 à 5, caractéristique en ce que la pièce de soupape (261) comprend un disque d'étanchéité (265), qui est muni de la surface d'étanchéité (267).

7. Pompe à piston suivant l'une des revendications 1 à 6, caractérisée en ce que la pièce de soupape (261) est logée dans une chambre de refoulement (259) qui est cylindrique.

8. Pompe à piston suivant l'une des revendications 1 à 7, caractérisée en ce que la course a du piston (237) est plus grande que la distance b entre le côté frontal (269) du piston (237) et la surface d'étanchéité (267).

9. Pompe à piston suivant l'une des revendications 8, caractérisée en ce que la différence $\Delta b$ = (a − b) entre la course a et la distance b est de 5/100 mm.

10. Pompe à piston suivant l'une des revendications 1 à 9, caractérisée en ce que le piston (237) peut passer en sa position haute par un système électromagnétique (251) et peut être ramené à sa position de repos par un système à aimant permanent (221, 223, 234).

11. Pompe à piston suivant l'une des revendications 1 à 10, caractérisée par son montage dans un dispositif implantable de dosage de médicaments, notamment pour l'insuline.

12. Pompe à piston suivant l'une des revendications 1 à 11, caractérisée en ce qu'il règne dans son conduit d'admission (203) une pression plus basse que dans son conduit de refoulement (209).

13. Pompe à piston suivant l'une des revendications 3 à 12, caractérisée en ce qu'un diaphragme (293) est disposé entre la surface d'étanchéité (267) et le côté frontal (269) du piston.(237) (figure 3).

14. Pompe à piston suivant la revendication 13, caractérisée en ce que sur le côté frontal (269) du piston (237) est monté un organe de refoulement (270), qui s'adapte dans l'ouverture (297) du diaphragme (293) (figure 3).

FIG 1

EP 0 287 920 B1

FIG 2

FIG 3